# EUROPÄISCHE PATENTSCHRIFT

(11) **EP 1 962 737 B1**
(45) Veröffentlichungstag und Bekanntmachung des Hinweises auf die Patenterteilung: **10.06.2009**
(21) Anmeldenummer: 06828626.9
(22) Anmeldetag: 07.12.2006
(51) Int. Cl.: A61F 2/68, A61F 2/58

(54) **HANDPROTHESE MIT ZWEI ANTRIEBSVORRICHTUNGEN**
HAND PROSTHESIS
PROTHESE DE MAIN

(30) Priorität: 20.12.2005 DE 102005061312
(43) Veröffentlichungstag der Anmeldung: 03.09.2008
(73) Patentinhaber: Otto Bock HealthCare GmbH, 37115 Duderstadt (DE)
(72) Erfinder: PUCHHAMMER, Gregor, 1130 Wien (AT)
(74) Vertreter: Stornebel, Kai
(86) Internationale Anmeldenummer: PCT/DE2006/002176
(87) Internationale Veröffentlichungsnummer: WO 2007/076764

(56) Entgegenhaltungen:
- EP-A1- 0 045 818
- EP-A1- 1 195 151
- WO-A-03/017876
- GB-A- 1 201 182
- GB-A- 1 585 256
- US-A- 5 080 682
- US-A- 5 888 246
- US-A1- 2003 195 638

## Beschreibung

Die Erfindung betrifft eine Handprothese mit einem Chassis, an dem zumindest eine Fingerprothese gelenkig gelagert ist, die über einen Antrieb um zumindest eine Schwenkachse bewegbar ist.

Wenn eine Hand amputiert werden musste oder durch einen Unfall irreversibel von einem Arm abgetrennt wurde, kann die Optik und teilweise die Funktion der Hand durch eine Handprothese ersetzt werden. Dazu muss die Handprothese in der lage sein, Greifeinrichtungen, die als Fingernachbildungen ausgebildet sein können, zueinander zu verlagern, um ein Greifen eines Gegenstandes zu ermöglichten.

Neben einem Zweifingergreifer, wie er aus der US 2003/00195638 A1 bekannt ist, werden Handprothesen vorgeschlagen, die über einen Antrieb verfügen, der über ein Kegelradgetriebe starr mit einem Handchassis verbunden ist. Je nach Drehrichtung des Antriebes werden dabei die Fingerprothesen aufeinander zu oder voneinander weg bewegt. Dieser Antrieb kann über myoelektrische Signale angesteuert werden. Eine solche Handprothese ist in der US 2005/0021154 A1 beschrieben. Die Anmutung einer solchen Handprothese ist wenig natürlich.

Aus der US 5,888,246 ist eine Fingerprothese mit einem integrierten Motor mit Getriebe bekannt, bei der über eine Schnecke eine Verschwenkung um ein Zahnrad bewirkt wird.

Die US 5,080,682 betrifft eine Prothese mit einem Chassis, an dem eine Fingerprothese gelenkig gelagert ist, die über einen Antrieb um zumindest eine Schwenkachse relativ zu dem Chassis schwenkbar gelagert ist, wobei ein erster Antrieb in dem Chassis angeordnet und über eine Kraftübertragungseinrichtung mit der Fingerprothese gekoppelt ist, so dass eine Verschwenkung um eine erste Schwenkachse ermöglicht wird. Für den Damen ist ein weiterer Antrieb vorgesehen, der ebenfalls an dem Chassis gelagert ist. Dieser zweite Antrieb verschwenkt einen Träger für die Fingerglieder, so dass eine Verschwenkung zumindest eines Teils der Fingerprothese um eine zweite Schwenkachse relativ zu der ersten Schwenkachse ermöglicht ist.

Aufgabe der vorliegenden Erfindung ist es, eine Handprothese bereitzustellen, die eine dem natürlichen Erscheinungsbild einer Hand angenäherte Gestaltung und eine verbesserte Funktionatität aufweist.

Erfindungsgemäß wird diese Aufgabe durch eine Handprothese mit den Merkmalen des Anspruchs 1 gelöst. Vorteilhafte Ausgestaltungen und Weiterbildungen der Erfindung sind in den Unteransprüchen beschrieben.

Die erfindungsgemäße Handprothese mit einem Chassis, an dem zumindest eine Fingerprothese gelenkig gelagert ist, die über einen Antrieb, der mit der Fingerprothese über eine Kraftübertragungseinrichtung verbunden ist, um zumindest eine Schwenkachse bewegbar ist, sieht vor, dass ein erster Antrieb in dem Chassis angeordnet und über die Kraftübertragungseinrichtung mit der Fingerprothese gekoppelt ist, der eine Verschwenkung um eine erste Schwenkachse ermöglicht. Weiterhin ist ein zweiter Antrieb in der Fingerprothese angeordnet, der eine Verschwenkung zumindest eines Teiles der Fingerprothese um eine zweite Schwenkachsen relativ zu der ersten Schwenkachse ermöglicht. Dadurch ist es möglich, eine komplex Bewegung der Finger sehr gut nachzubilden, ohne dass die Handprothese als solche sehr groß wird oder eine komplizierte Mechanik benötigt wird, mit der von einem zentralen Antrieb aus die unterschiedlichen Bewegungen eingeleitet werden. Bevorzugt sind die Antriebe voneinander entkoppelt, um unabhängig voneinander die jeweiligen Komponenten oder Prothesenteile ansteuern und verlagern zu können. Da der Bauraum für den zweiten Antrieb beschränkt ist, werden bevorzugt schnelllaufende, kleinbauende Motoren eingesetzt. Um eine ausreichende Verstellkraft bereitstellen zu können, weist der zweite Antrieb ein Getriebe auf, insbesondere ein Schrägschraubgetriebe, das mit einem Abtriebselement gekoppelt ist. Zwischen dem schneillaufenden Antrieb und dem Abtriebselement findet bereits eine erste Untersetzung statt, so dass das Abtriebselement mit einer wesentlich geringeren Drehzahl als der Antrieb dreht. Dadurch ist es möglich, neben einer Drehrichtungsumtenkung auch ein höheres Antriebsmoment bereitzustellen. Das Abtriebselement selbst ist als Schnecke ausgebildet, die in ein Zahnradsegment eingreift und dieses kämmt, so dass das Abtriebselement mit dem Zahnradsegment ein Schneckengetriebe ausbilden. Um eine ungewollte Verlagerung des zweiten Antriebes mitsamt dem Schneckengetriebe um die zweite Verschwenkachse zu vermeiden, ist dieses Schneckengetriebe selbsthemmend ausgebildet.

Eine Weiterbildung der Erfindung sieht vor, dass die Kraftübertragungseinrichtung zwischen dem Antrieb und der Fingerprothese drucknachgiebig oder biegeelastisch und zugstarr ausgebildet ist. Während die herkömmlichen Handprothesen eine starre Kopplung zwischen Antrieb und Fingerprothese vorsehen, ist durch die drucknachgiebige oder biegeelastische Kopplung des Antriebes mit der Fingerprothese gewährleistet, dass auf Druckbelastung der Kraftübertragungseinrichtung, also bei einer Krafteinwirkung, die ein Schließen der Hand bzw. einer Verkleinerung des Winkels zwischen der Fingerprothese und dem Handchassis bewirkt, die Fingerprothese nachgibt und neben einer natürlicheren Anmutung auch eine Schonung der mechanischen Komponenten bewirkt. Die Schonung der Komponenten ist dadurch begründet, dass die mitunter erheblichen Kräfte, die bei einem zufälligen Anstoßen der Fingerprothesen an Gegenstände entstehen, nicht unmittelbar über die Kraftübertragungseinrichtung auf den Antrieb übertragen werden. Vielmehr werden aufgrund der biegeelastischen, vorzugsweise rückfedernden Ausgestaltung der Kraftübertragungseinrichtung eine Verlagerung und eine Umwandlung der auf die Fingerprothese einwirkenden Kräfte in eine Bewegung ermöglicht. Diese Bewegung kann bis zum maximalen Beugewinkel der Fingerprothese erfolgen.

Um dagegen eine ungehinderte und zuverlässige sowie präzise Schließbewegung der Fingerprothese, ausgehend von einer geöffneten Grundstellung, sicherzustellen, ist die Kraftübertragungseinrichtung zugstarr ausgebildet, das heißt, dass Zugkräfte möglichst ohne Dehnung der Kraftübertragungseinrichtung übertragen werden. Dazu ist es vorgesehen, dass die Kraftübertragungseinrichtung bevorzugt eine Seil-, Litzen- oder Faserkomponente aufweist, über die die Zugkräfte übertragen werden. Diese Seil-, Litzen- oder Faserkomponente kann aus einem Drahtseil oder hochfesten Fasern wie Kohlefasern, Aramid oder Glasfasern oder Naturfasern hergestellt sein. Daneben können andere Kunststofffasern Einsatz finden. Damit hohe Zugkräfte von dem Antrieb auf die Fingerprothese übertragen werden können, ist die Seil-, Litzen- oder Faserkomponente als eine geschlossene, offene oder verdrehte Schlaufe ausgebildet.

Eine Weiterbildung der Erfindung sieht vor, dass die Kraftübertragungseinrichtung eine Elastomerkomponente aufweist, durch die es möglich ist, die Biegeelastizität der Kraftübertragungseinrichtung über einen weiten Bereich einzustellen. Durch die geeignete Wahl eines Elastomerwerkstoffes, der bevorzugt die Seil- oder Faserkomponente zumindest teilweise umschließt oder vollständig aufnimmt, ist es möglich, einen in Ruhelage formstabilen Körper herzustellen, der einerseits aufgrund der eingelagerten Seil- oder. Faserkomponente sehr hohe Zugkräfte und andererseits aufgrund der Elastomerkomponente ein gewünschtes biegeelastisches und druckelastisches Verhalten aufweist.

Alternativ kann die Kraftübertragungseinrichtung als eine Feder-DämpferEinheit ausgestaltet sein, insbesondere als eine Pneumatikeinheit, bei der auf Druckbelastung ein Luftvolumen komprimiert wird, das sich nach Wegfall der Druckkraft entspannt und eine Rückverlagerung eines Pneumatikkolbens und damit der Fingerprothese bewirkt.

Zur Ankopplung der Kraftübertragungseinrichtung an den Antrieb und die Fingerprothese sind Lagerbuchsen vorgesehen, die in der Kraftübertragungseinrichtung eingebettet sind. Bevorzugt werden diese Lagerbuchsen von dem Elastomerelement bzw. der Elastomerkomponente umschlossen und befinden sich innerhalb der Seil- oder Faserkomponente, bevorzugt innerhalb der Seil- oder Faserschlaufe.

Um eine Rückstellbewegung einer in Richtung Handchassisinnenfläche gebogenen Fingerprothese realisieren zu können, ist die Kraftübertragungseinrichtung federelastisch ausgebildet, so dass die Fingerprothese ohne Beaufschlagung mit einer Zugkraft durch den Antrieb oder durch ein mit dem Antrieb gekoppeltes Übertragungselement in eine Ausgangsstellung zurückbewegt wird. Diese Ausgangsstellung entspricht bevorzugt einer leicht geöffneten Hand. Die Kraftübertragungseinrichtung ist somit in der Lage, eine -begrenzte Druckkraft zu übertragen. Durch eine entsprechende Anlenkung oder Ausbildung der Kraftübertragungseinrichtung kann eine leichte Überstreckung der Fingerprothesen, ausgehend von der Grundstellung, ermöglicht werden. Die Federrate der Kraftübertragungseinrichtung ist dabei so bemessen, dass bei einer Beaufschlagung der Kraftübertragungseinrichtung mit einer Druckkraft, also bei einem Verschwenken der Fingerprothese in Richtung Handchassisinnenfläche, eine Rückstellung der Fingerprothese in die Ausgangsstellung bewirkt wird. Die Rückstellkraft muss daher so groß sein, dass die Halte- und Reibungskräfte innerhalb der Handprothese überwunden werden.

Die Kraftübertragungseinrichtung zur Übertragung von Kräften von einem Antrieb einer Handprothese auf gelenkig an einem Handchassis gelagerten Fingerprothese ist wie oben beschrieben ausgebildet und ermöglicht eine leichte und preiswerte Kopplung von Antrieb und Fingerprothese sowie eine wirksame Übertragung von Zugkräften. Darüber hinaus wird eine entgegen einer ungewollten Belastung nachgiebige Lagerung und eine einfache Rückstellung mit der Kraftübertragungseinrichtung erreicht.

Die Antriebe können als Elektromotoren ausgebildet sein, wobei der erste Antrieb als ein relativ großer, langsam laufende Flachmotor ausgebildet ist der für die Verlagerung der Fingerprothese relativ zu dem Handchassis eingesetzt wird. Die in den jeweiligen Fingerprothesen angeordneten zweiten Antriebsmotoren sind für eine Fingerkrümmung oder für eine zur Handinnenfläche gerichtete Bewegung vorgesehen.

Um ausreichend Raum für die Unterbringung des zweiten Antriebs zu haben, ist dieser bevorzugt in dem Bereich des Phalanx Proximalis eines natürlichen Fingers angeordnet, wobei eine bevorzugte Anwendung in einer Daumenprothese zu sehen ist, da das Daumengrundglied dicker als die übrigen Grundglieder der übrigen Finger sind und so mehr Raum für die Unterbringung des Antriebes zur Verfügung seht.

Um eine zusammengesetzte Bewegung der bevorzugt winklig zueinander ausgerichteten ersten und zweiten Verschwenkachsen ermöglichen zu können, ist der Zweitantrieb schwenkbar an dem Zahnradsegment gelagert, das wiederum schwenkbar an dem Chassis um die erste Verschwenkachse gelagert ist. Somit kann die Fingerprothese um zwei Achsen verschwenkt werden, wobei einmal der komplette Antrieb mitsamt Schneckengetriebe und Zahnradsegment um eine erste Schwenkachse verschwenkbar ist, während zur Realisierung der zweiten Bewegung lediglich eine Verschwenkung um eine Achse an dem Zahnradelement vorgesehen ist. Die Winkelstellung der Achsen zueinander kann durch die Verschwenkung aufgrund des zweiten Antriebes verändert werden, um entweder einen Lateralgriff, eine Neutralposition ohne Greiffunktion, die optisch einen natürlichen Eindruck hinterlässt, oder einen Spitzgriff der Handprothese zu realisieren.

Nachfolgend wird ein Ausführungsbeispiel der Erfindung anhand der beigefügten Figuren näher erläutert. Gleiche Bezugszeichen in den unterschiedlichen Figuren bezeichnen gleiche Elemente. Es zeigen:
- Figur 1-: eine schematische Darstellung einer Handprothese;
- Figur 2-: eine schematische Teildarstellung des Funktionsaufbaus einer Handprothese;
- Figuren 3a - 3c -: eine Kraftübertragungseinrichtung in unterschiedlichen Ansichten; sowie
- Figur 4 -: ein Funktionsaufbau einer Fingerprothese.

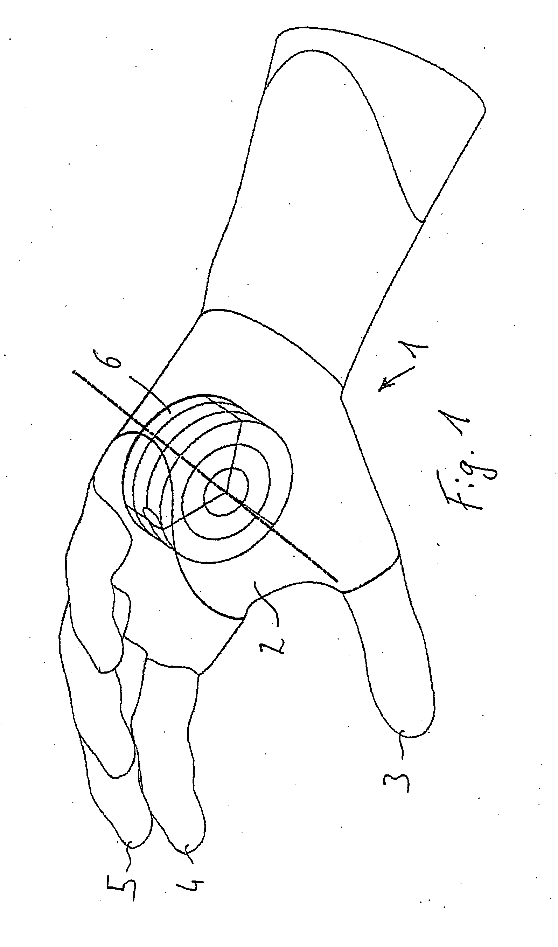

In der Figur 1 ist eine Handprothese 1, bestehend aus einem Handchassis 2 und zumindest drei gelenkig an dem Handchassis 2 gelagerten Fingerprothesen 3, 4, 5 gezeigt. Die Fingerprothesen 3, 4, 5 entsprechen dem Daumen, Zeigefinger und Mittelfinger einer natürlichen Hand. Eine bewegliche und über einen Antrieb 6 aktuierbare Lagerung dieser drei Fingerprothesen 3, 4, 5 reicht aus, um die Mehrzahl der Greifverrichtungen einer Hand ausführen zu können. Die beiden übrigen Finger, der Ringfinger und der kleine Finger, können passiv mitbewegt werden und aus einem Elastomermaterial bestehen, um eine möglichst natürliche Anmutung zu erzielen. Innerhalb des Handchassis 2 ist der Antrieb 6 in Gestalt eines elektrischen Antriebes, z.B. eines Flachläufermotors, mit einem dazugehörigen Getriebe gelagert. Ebenfalls kann eine nicht dargestellte Energiequelle für den Antrieb 6 innerhalb des Handchassis 2 angeordnet sein. Eine Ansteuerung des Antriebs 6 erfolgt über ein Steuergerät, das ebenfalls in dem Handchassis 2 angeordnet sein kann. Die entsprechenden Signale können über eine Fernbedienung generiert oder als myoelektrische Signale ausgebildet sein.

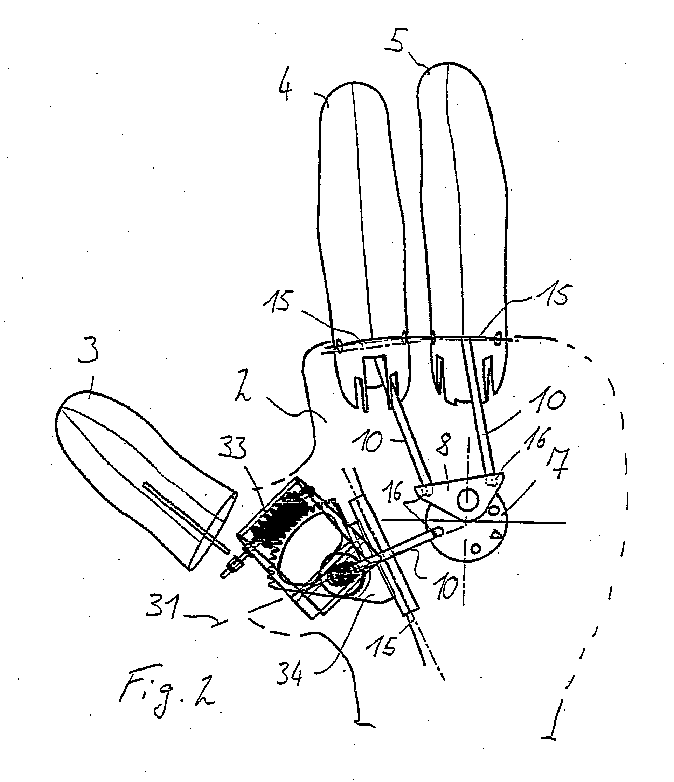

In der Figur 2 ist eine schematische Darstellung der Funktionsweise der Handprothese 1 gezeigt. An dem Handchassis 2 sind die drei Fingerprothesen 3, 4, 5 um Gelenkachsen 15 verschwenkbar gelagert. Die Fingerprothesen 3, 4, 5 sind über Kraftübertragungseinrichtungen 10, deren Aufbau weiter unten detailliert beschrieben werden wird, mit einer Drehscheibe 7 verbunden, die von dem Elektromotor 6 angetrieben wird. Die Kraftübertragungseinrichtungen 10 sind an der Drehscheibe 7 auf Achsen 16 entweder direkt oder über eine Wippe 8 gelagert. Der Zeigefinger 4 und der Ringfinger 5 sind über die Wippe 8, die drehbar auf der Drehscheibe 7 gelagert ist, miteinander gekoppelt. Die Drehscheibe 7 selbst ist entweder unmittelbar auf einer Abtriebswelle des Antriebes 6 oder einer Getriebeausgangswelle gelagert. Wird der Antrieb 6 aktiviert, wird die Drehscheibe 7 um einen entsprechenden Drehwinkel bewegt. Dadurch verlagern sich die Achsen 16 relativ zu den Schwenkachsen 15 der Fingerprothesen 3, was aufgrund der zugstarren Ausbildung der Kraftübertragungseinrichtungen 10 und einer von den Drehachsen 15 beabstandeten Anlenkung der Kraftübertragungseinrichtungen 10 an den Fingerprothesen 3, 4, 5 zu einer Verschwenkung der Fingerprothesen 3, 4, 5 führt. Wird der Antrieb 6 reversiert und bewegt sich die Drehscheibe 7 in eine Position, in der die Achsen 16 eine minimale Entfernung zu den Schwenkachsen 15 der Fingerprothesen 3, 4, 5 aufweisen, ist die geöffnete Ausgangsstellung erreicht. Durch die federelastischen Eigenschaften der Kraftübertragungseinrichtungen 10 werden dann die Fingerprothesen 3, 4, 5 in ihre geöffnete Ausgangsstellung bewegt. Dabei ist es vorgesehen, dass die Kraftübertragungseinrichtungen 10 wesentlich höhere Zugkräfte als Druckkräfte übertragen können. Dies entspricht den physiologischen Gegebenheiten einer natürlichen Hand, die beim Schließen der Hand wesentlich größere Kräfte als beim Öffnen aufbringen kann. Aus Gründen der Übersichtlichkeit sind der Ringfinger und der kleine Finger nicht dargestellt, diese können passiv an dem Mittelfinger 5 angelenkt und dadurch mitbewegt werden.

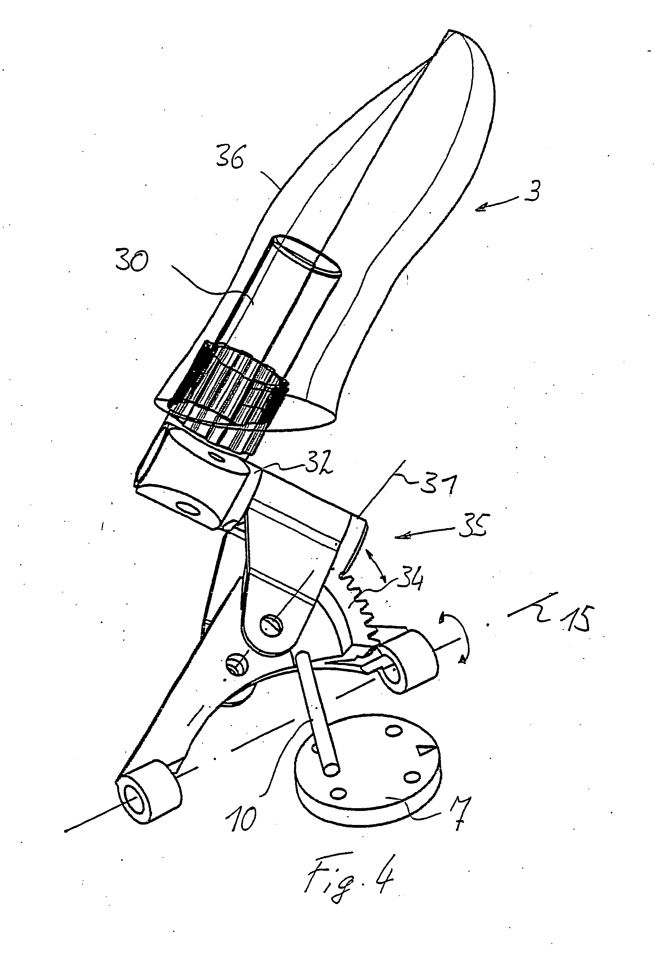

In der Figur 2 ist weiterhin zu erkennen, dass die Daumenprothese 3 neben der ersten Schwenkachse 15 eine zweite Schwenkachse 31 aufweist, um die zumindest das distale Ende der Daumenprothese 3 verschwenkbar gelagert ist. Über einen nur in der Figur 4 dargestellten zweiten Antrieb 30 und ein Schrägschraubgetriebe 32 wird eine Abtriebsschnecke 33 bewegt, die mit einem Zahnradsegment 34 kämmt und so eine Verlagerung der Fingerprothese 3 mit dem Antrieb 30 und dem Getriebe 32 um die Schwenkachse 31 bewirkt. Werden beide Antriebe 6, 30 gleichzeitig aktiviert, wird entsprechend der Verlagerungsgeschwindigkeiten eine Kombinationsbewegung der Daumenprothese 3 palmar und ulnar ausgeführt, was einer natürlichen Daumenbeweglichkeit entspricht.

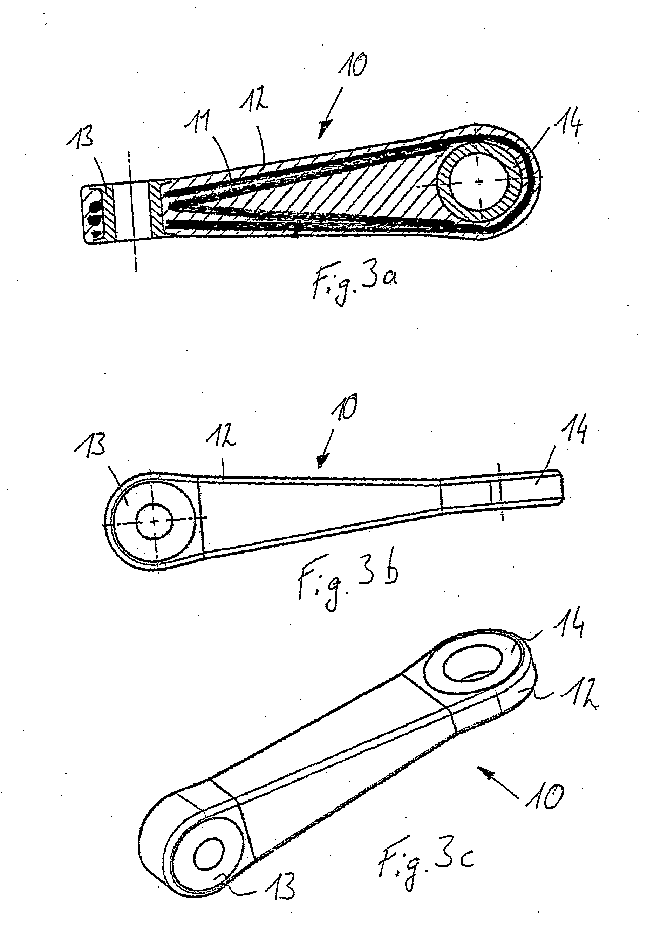

In der Figur 3 ist in einer Schnittdarstellung eine Kraftübertragungseinrichtung 10. gezeigt. Diese besteht aus einer Seil- oder Faserkomponente 11, die vorliegend als eine Schlaufe ausgebildet ist. Die Seilkomponente 11 kann aus mehreren Litzen oder Einzelschlaufen bestehen und als Stahlseil oder Kunststoffseil oder aus einem anderen, hochfesten Faserwerkstoff bestehen. Die Seilkomponente 11 ist in einem Elastomerelement 12 eingebettet, wodurch die Kraftübertragungseinrichtung 10. eine formstabile, jedoch biegeelastische Gestalt erhält. Die Elastomerkomponente 12 kann aus einem Silikon, einem Kautschuk oder einem anderen, elastischen Werkstoff bestehen. Trotz der Formstabilität ist durch die Biegsamkeit der Seil- oder Faserkomponente 11 und dem druckelastischen oder biegeelastischen Verhalten der Elastomerkomponente 12 eine Verformung, insbesondere Biegung aufgrund auf die Kraftübertragungseinrichtung 10 einwirkender Druckkräfte möglich. Dadurch können die über die Kraftübertragungseinrichtung 10 mit dem Antrieb 6 bzw. der Drehscheibe 7 gekoppelten Fingerprothesen 3, 4, 5 in Richtung auf die Handchassisinnenfläche verlagert werden, wobei eine Rückverlagerung aufgrund des federelastischen Verhaltens der Kraftübertragungseinrichtungen 10 erfolgt, wenn die entsprechende Gegenkraft entfällt.

Innerhalb der als Schlaufe ausgebildeten Seil- oder Faserkomponente 11 sind zwei Lagerbuchsen 13, 14 angeordnet, die ebenfalls in der Elastomerkomponente 12, beispielsweise einem Silikonwerkstoff, eingebettet sind. Die Lagerbuchen 13, 14 werden auf entsprechenden Achsen an den Fingerprothesen 3, 4, 5 und auf Achsen 16 an der Drehscheibe 7 bzw. der Brücke 8 gelagert. Die Lagerbuchsen 13, 14 bestehen beispielsweise aus Bronze, um eine Gleitlagerung mit den entsprechenden Achsen 16 auszubilden. Aus Gründen der Übersichtlichkeit sind die Kopplungsachsen an den Fingerprothesen 3, 4, 5 nicht dargestellt. Diese Kopplungsachsen liegen beabstandet zu den Drehachsen 15, so dass durch Aufbringung von Zugkräften über die Kraftübertragungseinrichtungen 10 ein Moment um die Drehachsen 15 erzeugt wird, was zu einer korrespondierenden Verlagerung der Fingerprothesen 3, 4, 5 führt.

In den Figuren 3b und 3c ist zu erkennen, dass die Drehachsen der Lagerbuchen 13, 14 senkrecht zueinander stehen, was in der konkreten Anordnung der Drehscheibe 7 und der darauf angeordneten oder ihr zugeordneten Achsen 16 begründet ist. Die Drehachsen der Lagerbuchsen 13, 14 können auch parallel oder in einem anderen Winkel zueinander ausgerichtet sein.

Ebenfalls ist in den Figuren 3a bis 3c zu erkennen, dass die Seil- oder Faserkomponente 11 vollständig in dem Elastomer 12 eingebettet sind, wodurch einerseits die Seil- oder Faserkomponente 11 vor äußeren Einflüssen geschützt und andererseits die Formstabilität der Kraftübertragungseinrichtung 10 erhöht wird.

Alternativ zu der dargestellten Ausführungsform kann die Kraftübertragungseinrichtung 10 auch aus einem anderen, drucknachgiebigen, z.B. federelastischen und zugstarren Element hergestellt sein, beispielsweise einem federnden Knick- oder Auslenkstab oder einer entsprechend konstruierten Drahtschlaufe.

Durch die druckelastische Lagerung findet keine unmittelbare Übertragung von Stoßkräften über die Fingerprothesen 3, 4. 5 auf den Antrieb 6 bzw. die Drehscheibe 7 statt. Vielmehr werden unbeabsichtigte Anstoßbewegungen abgefedert und gedämpft. Dies erhöht neben einer natürlichen Anmutung der Handprothese 1 auch die Lebensdauer der Lagerungen und Antriebskomponenten, beispielsweise bei einem Sturz.

Alternativ zu der dargestellten Ausführungsform kann die Kraftübertragungseinrichtung auch als eine Feder-Dämpfereinheit mit einer entsprechenden Steuerung ausgestattet sein, beispielsweise über einen pneumatischen oder hydraulischen Zylinder mit entsprechender Ventilsteuerung, die Zugkräfte wirksam übertragen können, bei Druckkräften jedoch eine elastische Nachgiebigkeit vorsehen. Durch eine pneumatische Ausgestaltung wird eine Rückverlagerung der nach innen gebogenen Fingerprothesen bewirkt.

Die Elastomerkomponente kann bei einer ausreichend biegeelastischen Ausgestaltung der Seilkomponente weggelassen werden; bei einer ausreichenden Zugfestigkeit der Elastomerkomponente kann diese als alleinige Kraftübertragungseinrichtung ausgebildet sein.

In der Figur 4 ist eine Detaildarstellung des Funktionsaufbaues der Daumenprothese 3 gezeigt, mit einem Formkörper 36, der der Kontur eines natürlichen Daumens nachgebildet ist. Innerhalb des Formkörpers 36, der als Hohlkörper ausgebildet ist, ist ein Freiraum, in dem der zweite Antrieb 30 angeordnet und befestigt ist. Der Formkörper 36 ist somit an den Antrieb 30 gekoppelt, beispielsweise angeklebt oder festgeklemmt. Der Antrieb 30 ist über ein Winkelgetriebe in Gestalt eines Schrägschraubgetriebes 32 und der in der Figur 2 beschriebenen Schnecke 33 mit dem Zahnradsegment 34 gekoppelt. Bei Aktivierung des Antriebes 30 wird die Schnecke 33 in die eine oder andere Richtung gedreht. Aufgrund der schwenkbaren Lagerung um die Drehachse 31 an dem Zahnradsegment 34 ist eine Bewegung um die Schwenkachse 31 in Richtung des Doppelpfeils möglich. Es kann dabei eine radiale oder ulnare Bewegung ausgeführt werden. Das Zahnradsegment 34 selbst ist schwenkbar um die erste Schwenkachse 15 gelagert und kann durch eine Drehung der Drehscheibe 7 und die dadurch bewirkte Verlagerung des Kraftübertragungselementes 10 palmar oder dorsal verschwenkt werden. Diese Verschwenkbewegung ist ebenfalls durch den Doppelpfeil um die Schwenkachse 15 angedeutet.

Der zweite Antrieb 30 ist ebenfalls ein elektrischer Antrieb, z.B. ein Elektromotor oder Piezomotor und befindet sich bevorzugt in der Längsachse zwischen dem Sattel- und dem Interphalangialgelenk. Aufgrund der kleinen Bauart und dem ggf. notwendigen, hohen Antriebsmoment ist der Antrieb 30 als Schnellläufer ausgebildet, wobei das Übersetzungsgetriebe 32 als Schrägschraubgetriebe ausgebildet sein kann und eine etwa rechtwinklige Umlenkung der Abtriebsachse relativ zu der Längsachse des zweiten Antriebs 30 erzeugt. Aufgrund dieser Abwinklung der Abtriebsachse ist es möglich, dass die Schnecke 33, die das Zahnradsegment 34 kämmt, eine entsprechende Daumenbewegung bewirkt. Alternativ zu einer im Wesentlichen rechtwinkeligen Umlenkung kann auch eine beliebige Winkelstellung durch eine entsprechende Ausgestaltung des Getriebes realisiert werden, beispielsweise ± 45° zu einer rechtwinkeligen Ausrichtung.

Der in dem Handchassis 2 angeordnete erste Antrieb 6 ist bevorzugt ein langsam laufender Flachläufermotor mit einem hohen Drehmoment, der mit einem hoch untersetzten Getriebe gekoppelt ist, um eine entsprechende langsame Greifbewegung ausführen zu können. Die Steuerungssignale können entweder von einer Fernsteuerung oder myoelektrische Signale und ein Steuergerät generiert werden. Über diesen ersten Antrieb 6 und die Drehscheibe 7 ist das Zahnradsegment 34 mitsamt der Schnecke 33 sowie dem Getriebe 32 und dem Antrieb 30, abgedeckt durch den Formkörper 36, verlagerbar.

## Patentansprüche

1. . Handprothese mit einem Chassis, an dem eine Fingerprothese gelenkig gelagert ist, die über einen Antrieb um zumindest eine Schwenkachse relativ zu dem Chassis bewegbar ist, wobei ein erster Antrieb (6) in dem Chassis (2) angeordnet und über eine Kraftübertragungseinrichtung (10) mit der Fingerprothese (3, 4, 5) gekoppelt ist, der eine Verschwenkung um eine erste Schwenkachse (15) ermöglicht, und dass ein zweiter Antrieb (30) vorgesehen ist, der eine Verschwenkung zumindest eines Teiles der Fingerprothese (3) um eine zweite Schwenkachse (31) relativ zu der ersten Schwenkachse (15) ermöglicht, **dadurch gekennzeichnet, dass** der zweite Antrieb (30) in der Fingerprothese (3) angeordnet und über ein Getriebe (32), mit sich nicht schneidenden Achsen, mit einem Abtriebselement (33) gekoppelt ist, wobei das Abtriebselement (33) als Schnecke ausgebildet ist, die ein Zahnradsegment (34) kämmt und ein Schneckengetriebe (35) ausbildet

2. . Handprothese nach Anspruch 1, **dadurch gekennzeichnet, dass** die Antriebe (30, 6) voneinander entkoppelt sind.

3. . Handprothese nach Anspruch 1 oder 2, **dadurch gekennzeichnet, dass** die Kraftübertragungseinrichtung (10) zugstarr und drucknachgibig oder biegeelastisch ausgebildet ist..

4. . Handprothese nach einem der voranstehenden Ansprüche, **dadurch gekennzeichnet, dass** die Kraftübertragungskomponente (10) eine Seil- oder Faserkomponente (11) oder Litze aufweist.

5. . Handprothese nach Anspruch 4, **dadurch gekennzeichnet, dass** die Seil- oder Faserkomponente (11) oder Litze als geschlossene, offene oder verdrehte Schlaufe ausgebildet ist.

6. . Handprothese nach einem der voranstehenden Ansprüche, **dadurch gekennzeichnet, dass** die Kraftübertragungseinrichtung (10) eine Elastomerkomponente (12) aufweist.

7. . Handprothese nach Anspruch 4 und 6, **Dadurch gekennzeichnet, dass** die Elastomerkomponente (12) die Seil- oder Faserkomponente (11) oder Litze zumindest teilweise umschließt.

8. . Handprothese nach einem der voranstehenden Ansprüche, **dadurch gekennzeichnet, dass** die Kraftübertragungseinrichtung (10) Lagerbuchsen (13. 14) zur Aufnahme von Achsen (15, 16) aufweist, die dem Handchassis (2) und der Fingerprothese (3, 4, 5) zugeordnet sind.

9. . Handprothese nach einem der voranstehende Ansprüche, **dadurch gekennzeichnet, dass** die Kraftübertragungseinrichtung (10) federelastisch ausgebildet ist.

10. . Handprothese nach Anspruch 9, **dadurch gekennzeichnet, dass** die Federrate der Kraftübertragungseinrichtung (10) so bemessen ist, dass bei einer Beaufschlagung der Kraftübertragungseinrichtung (10) mit einer Druckkraft eine Rückstellung der Fingerprothese (3, 4, 5) in eine Ausgangsstellung bewirkt wird.

11. . Handprothese nach einem der voranstehenden Ansprüche, **dadurch gekennzeichnet, dass** die Antriebe (30, 6) als Elektromotoren ausgebildet sind.

12. . Handprothese nach einem der voranstehenden Ansprüche, **dadurch gekennzeichnet, dass** der zweite Antrieb (30) in dem Bereich des Phalanx proximalis eines natürlichen Fingers, insbesondere Daumens angeordnet ist.

13. . Handprothese nach einem der voranstehenden Ansprüchen, **dadurch gekennzeichnet, dass** der zweite Antrieb (30) schwenkbar an dem Zahnradsegment (34) gelagert ist.

14. . Handprothese nach einem der voranstehenden Ansprüche, **dadurch gekennzeichnet, dass** das Schneckengetriebe (35) selbsthemmend ausgebildet ist.

15. . Handprothese nach einem der voranstehenden Ansprüche, **dadurch gekennzeichnet, dass** das Zahnradsegment (34) an dem Chassis (2) verschwenkbar gelagert ist.

16. . Handprothese nach einem der voranstehenden Ansprüche, **dadurch gekennzeichnet, dass** der zweite Antrieb (30) die Pingerprothese (3, 4, 5) zwischen mehreren festgelegten Positionen verfährt.

## Claims

1. Hand prosthesis comprising a chassis, to which a finger prosthesis is articulated, said finger prosthesis being movable relative to the chassis about at least one pivot axis by means of a drive, wherein a first drive (6) is arranged in the chassis (2) and is coupled by means of a force transmission device (10) to the finger prosthesis (3, 4, 5), and allows pivoting about a first pivot axis (15), and in that a second drive (30) is provided, which allows pivoting of at least one part of the finger prosthesis (3) about a second pivot axis (31) relative to the first pivot axis (15), **characterised in that** the second drive (30) is arranged in the finger prosthesis (3) and is coupled by means of a gear mechanism (32) with non-intersecting axes to an output element (33), the output element (33) being formed as a worm, which meshes with a gear wheel segment (34) and forms a worm gear mechanism (35).

2. Hand prosthesis according to claim 1, **characterised in that** the drives (30, 6) are decoupled from one another.

3. Hand prosthesis according to claim 1 or 2, **characterised in that** the force transmission device (10) is rigid under tension and yielding under pressure or resiliently flexible.

4. Hand prosthesis according to any one of the preceding claims, **characterised in that** the force transmission component (10) has a cable or fibre component (11) or litz wire.

5. Hand prosthesis according to claim 4, **characterised in that** the cable or fibre component (11) or litz wire is formed as a closed, open or twisted loop.

6. Hand prosthesis according to any one of the preceding claims, **characterised in that** the force transmission device (10) has an elastomer component (12).

7. Hand prosthesis according to claim 4 and 6, **characterised in that** the elastomer component (12) at least partially surrounds the cable or fibre component (11) or litz wire.

8. Hand prosthesis according to any one of the preceding claims, **characterised in that** the force transmission device (10) has bearing bushings (13, 14) to receive spindles (15, 16), which are associated with the hand chassis (2) and the finger prosthesis (3, 4, 5).

9. Hand prosthesis according to any one of the preceding claims, **characterised in that** the force transmission device (10) is resiliently elastic.

10. Hand prosthesis according to claim 9, **characterised in that** the spring rate of the force transmission device (10) is set such that when the force transmission device (10) is loaded by a pressure force, the finger prosthesis (3, 4, 5) is returned to a starting position.

11. Hand prosthesis according to any one of the preceding claims, **characterised in that** the drives (30, 6) are formed as electric motors.

12. Hand prosthesis according to any one of the preceding claims, **characterised in that** the second drive (30) is arranged in the region of the phalanx proximalis of a natural finger, in particular a thumb.

13. Hand prosthesis according to any one of the preceding claims, **characterised in that** the second drive (30) is pivotably mounted on the gear wheel segment (34).

14. Hand prosthesis according to any one of the preceding claims, **characterised in that** the worm gear mechanism (35) is self-locking.

15. Hand prosthesis according to any one of the preceding claims, **characterised in that** the gear wheel segment (34) is pivotably mounted on the chassis (2).

16. Hand prosthesis according to any one of the preceding claims, **characterised in that** the second drive (30) moves the finger prosthesis (3, 4, 5) between a plurality of fixed positions.

## Revendications

1. Prothèse de main avec un châssis, sur lequel est logé une prothèse de doigt articulée, qui peut être bougée relativement au châssis par un entraînement selon au moins un axe de basculement, où un premier entrainement (6) est disposé dans le châssis (2) et est accouplé avec la prothèse de doigt (3, 4, 5) par un dispositif de transmission d'effort (10), qui rend possible un pivotement autour d'un premier axe de pivotement (15), et où est prévu un deuxième entraînement (30) qui rend possible un basculement d'au moins une pièce de la prothèse de doigt (3) autour d'un deuxième axe de basculement (31) relativement au premier axe de basculement (15), **caractérisée en ce que** le deuxième entraînement (30) est disposé dans la prothèse de doigt (3) et est accouplé avec un élément réducteur (33) par un engrenage (32) d'axe non concourant, où l'élément réducteur (33) est réalisé sous la forme d'une vis sans fin qui parcourt une partie de roue dentée et réalise une transmission par vis sans fin (35).

2. Prothèse de main selon la revendication 1, **caractérisée en ce que** les entraînements (30, 6) sont découplés l'un de l'autre.

3. Prothèse de main selon l'une quelconque des revendications 1 ou 2, **caractérisée en ce que** le dispositif de transmission d'effort (10) est rigide en traction et déformable sous la pression ou élastique en flexion.

4. Prothèse de main selon l'une quelconque des revendications précédentes, **caractérisée en ce que** l'élément de transmission d'effort (10) présente un élément à base de fibre (11) ou de corde ou un cordon.

5. Prothèse de main selon la revendication 4, **caractérisée en ce que** l'élément à base de fibre (11) ou de corde ou le cordon est réalisé sous la forme de boucle ouverte ou torsadée.

6. Prothèse de main selon l'une quelconque des revendications précédentes, **caractérisée en ce que** le dispositif de transmission d'effort (10) présente un composant en élastomère (12).

7. Prothèse de main selon l'une quelconque des revendications 4 à 6, **caractérisée en ce que** le composant en élastomère (12) renferme l'élément à base de fibre (11) ou de corde ou le cordon au moins en partie.

8. Prothèse de main selon l'une quelconque des revendications précédentes, **caractérisée en ce que** le dispositif de transmission d'effort (10) présente des coussinets (13, 14) recevant les axes (15, 16), qui correspondent au châssis de main (2) et à la de doigt (3, 4, 5).

9. Prothèse de main selon l'une quelconque des revendications précédentes, **caractérisée en ce que** le dispositif de transmission d'effort (10) est réalisé sous une forme à déformation élastique.

10. Prothèse de main selon la revendication 9, **caractérisée en ce que** le coefficient d'élasticité du dispositif de transmission d'effort (10) est choisi de façon à ce que l'application sur le dispositif de transmission d'effort (10) d'une pression provoque un mouvement vers l'arrière de la prothèse de doigt (3, 4, 5).

11. Prothèse de main selon l'une quelconque des revendications précédentes, **caractérisée en ce que** les entraînements (30, 6) sont réalisés sous la forme de moteurs électriques.

12. Prothèse de main selon l'une quelconque des revendications précédentes, **caractérisée en ce que** le deuxième entraînement (30) est disposé dans la région de la *phalanx proximalis* d'un doigt naturel, et en particulier du pouce.

13. Prothèse de main selon l'une quelconque des revendications précédentes, **caractérisée en ce que** le deuxième entraînement (30) est logé de façon à pouvoir basculer sur la portion de roue dentée (34).

14. Prothèse de main selon l'une quelconque des revendications précédentes, **caractérisée en ce que** la transmission par vis sans fin (35) est réalisée sous une forme autobloquante.

15. Prothèse de main selon l'une quelconque des revendications précédentes, **caractérisée en ce que** la portion de roue dentée (34) est logée de façon pivotante sur le châssis (2).

16. Prothèse de main selon l'une quelconque des revendications précédentes, **caractérisée en ce que** le deuxième entraînement (30) commande la prothèse de doigt (3, 4, 5) entre plusieurs positions définies.
